(19) Europäisches Patentamt
European Patent Office
Office européen des brevets

(11) EP 4 345 129 A1

(12) **EUROPÄISCHE PATENTANMELDUNG**

(43) Veröffentlichungstag:
**03.04.2024 Patentblatt 2024/14**

(21) Anmeldenummer: **23195912.3**

(22) Anmeldetag: **07.09.2023**

(51) Internationale Patentklassifikation (IPC):
***C08K 5/00*** *(2006.01)* ***C08K 5/11*** *(2006.01)*
***C08K 5/1515*** *(2006.01)* ***C07C 67/02*** *(2006.01)*
***C07C 67/03*** *(2006.01)* ***C07C 67/08*** *(2006.01)*
***C07C 69/34*** *(2006.01)*

(52) Gemeinsame Patentklassifikation (CPC):
(C-Sets verfügbar)
**C08K 5/11; C07C 67/03; C07C 67/08; C07C 69/34; C08K 5/0016; C08K 5/1515** (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **30.09.2022 EP 22199108**

(71) Anmelder: **Evonik Oxeno GmbH & Co. KG
45772 Marl (DE)**

(72) Erfinder:
- **Kraft, Johannes
63067 Offenbach (DE)**
- **Schulz, Imke
59348 Lüdinghausen (DE)**
- **Grass, Michael
45721 Haltern am See (DE)**

(74) Vertreter: **Evonik Patent Association
c/o Evonik Industries AG
IP Management
Bau 1042A/PB 15
Paul-Baumann-Straße 1
45772 Marl (DE)**

# (54) TETRAISOPENTYLESTER DER BUTANTETRACARBONSÄURE, DEREN HERSTELLUNG UND DEREN VERWENDUNG ALS WEICHMACHER

(57) Gegenstand der Erfindung sind Tetraisopentylester der Butantetracarbonsäure, ihre Herstellung und ihre Verwendung als Weichmacher oder als Teil einer Weichmacherzusammensetzung für Polymere. Weiterhin werden Weichmacherzusammensetzungen, die die Tetraisopentylester der Butantetracarbonsäure enthalten, und Kunststoffzusammensetzungen, die die Tetraisopentylester der Butantetracarbonsäure enthalten, offenbart.

EP 4 345 129 A1

(52) Gemeinsame Patentklassifikation (CPC): (Forts.)

C-Sets
**C07C 67/03, C07C 69/34;**
**C07C 67/08, C07C 69/34;**
**C08K 5/11, C08L 27/06;**
**C08K 5/1515, C08L 27/06**

**Beschreibung**

Die vorliegende Erfindung betrifft Tetraisopentylesterder Butantetracarbonsäure, ihre Herstellung und ihre Verwendung als Weichmacher oder als Teil einer Weichmacherzusammensetzung für Polymere. Weiterhin werden Weichmacherzusammensetzungen, die die Tetraisopentylester der Butantetracarbonsäure enthalten, und Kunststoffzusammensetzungen, die die Tetraisopentylester der Butantetracarbonsäure enthalten, offenbart.

Kunststoffen bzw. Polymeren werden in vielen Anwendungsgebieten Weichmacher oder Weichmacherzusammensetzungen zugesetzt, um die Verarbeitbarkeit zu verbessern und/oder um anwendungsrelevante Eigenschaften anzupassen. Aufgrund ihrer vorteilhaften Eigenschaften gehören noch immer Verbindungen aus der Gruppe der Phthalate, insbesondere Diethylhexylphthalat (DEHP), Diisononylphthalat (DINP) und Diisodecylphthalat (DIDP) zu den wichtigsten Weichmachern für Kunststoffe bzw. Polymere, insbesondere für PVC und Vinylchloridhaltige Copolymere. Auf Grund von Diskussionen über eventuelle toxikologische Effekte dieser Stoffklasse wird seit vielen Jahren nach Alternativen für Phthalat-basierte Weichmacher gesucht.

Als alternative Weichmacher werden verschiedene Verbindungen diskutiert, unter anderem auch Alkylester der Butantetracarbonsäure, die dem Fachmann beispielswiese bereits aus der US 2011/0257317 A1 bekannt sind. Die dort offenbarten Alkylester der Butantetracarbonsäure weisen einen maximalen Anteil von an der beta-Position verzweigten Alkylresten von 40% auf.

Im Hinblick auf Tetraisopentylester der Butantetracarbonsäure konnte nun überraschenderweise herausgefunden werden, dass, bezogen auf die gesamten isomeren Pentylreste, bei einem bestimmten molaren Anteil von 2-Methylbutylresten verbesserte Eigenschaften auftreten. Die zugrundliegende Aufgabe der vorliegenden Erfindung war deshalb die Bereitstellung von Tetraisopentylestern der Butantetracarbonsäure mit verbesserten Eigenschaften.

Gelöst wird die zugrundeliegende Aufgabe durch die Mischung von Tetraisopentylestern der 1,2,3,4-Butantetracarbonsäure gemäß Anspruch 1. Bevorzugte Ausführungsformen sind in den Unteransprüchen angegeben.

Im Rahmen der vorliegenden Erfindung werden die Begriffe "isopentyl", "Iso-Pentylreste" oder ähnliches verwendet. Damit wird unterstrichen, dass die erfindungsgemäßen Ester verschiedene Pentyl-Isomere, insbesondere 2-Methylbutyl und/oder 3-Methylbutyl und/oder n-Pentyl, enthalten. Die Begriffe isopentyl", "Iso-Pentylreste" oder ähnliches sind also Synonym zu dem Begriff "Pentyl" zu verstehen und weist nicht auf eine einzige spezielle C5-Alkylgruppe hin.

Die vorliegende Erfindung betrifft demnach eine Mischung von Tetraisopentylestern der 1,2,3,4-Butantetracarbonsäure, wobei 45 bis 60 Mol% der iso-Pentylreste in der Mischung 2-Methylbutylreste sind. Die Prozentangabe bezieht sich jeweils auf die gesamten iso-Pentylreste in der Mischung. In einer bevorzugten Ausführungsform der vorliegenden Erfindung sind in der Mischung von Tetraisopentylestern der 1,2,3,4-Butantetracarbonsäure 50 bis 60 Mol% der iso-Pentylreste in der Mischung 2-Methylbutylreste.

Die restlichen iso-Pentylreste in der erfindungsgemäßen Mischung von Tetraisopentylestern der 1,2,3,4-Butantetracarbonsäure sind vorzugsweise n-Pentylreste oder 3-Methylbutylreste, insbesondere sind 40 bis 55 Mol% der iso-Pentylreste in der erfindungsgemäßen Mischung n-Pentylreste, 3-Methylbutylreste oder eine Mischung aus n-Pentylresten und 3-Methylbutylresten sind. In einer bevorzugten Ausführungsform der vorliegenden Erfindung sind in der Mischung von Tetraisopentylestern der 1,2,3,4-Butantetracarbonsäure 40 bis 50 Mol% der iso-Pentylreste n-Pentylreste, 3-Methylbutylreste oder eine Mischung aus n-Pentylresten und 3-Methylbutylresten. In einer bevorzugten Ausführungsform der vorliegenden Erfindung sind in der erfindungsgemäßen Mischung von Tetraisopentylestern der 1,2,3,4-Butantetracarbonsäure 0 bis 4 Mol%, vorzugsweise 0 bis 2 Mol%, besonders bevorzugt 0- 1 Mol% der iso-Pentylreste 3-Methylbutylreste,

Die erfindungsgemäße Mischung von Tetraisopentylestern der 1,2,3,4-Butantetracarbonsäure kann durch Veresterung der 1,2,3,4-Butantetracarbonsäure mit einer geeigneten Isopentanolmischung hergestellt werden. Isopentanolmischung ist in diesem Zusammenhang so zu verstehen, dass diese Mischung zumindest 2-Methylbutanol und n-Pentanol und/oder 3-Methylbutanol enthält. Bei der Veresterung sollte verständlicherweise darauf geachtet werden, dass die Mischung ausreichend 2-Methylbutanol enthält, um die beanspruchte Menge an 2-Methylbutylresten im gebildeten Ester zu erhalten. Die Auswahl geeigneter Isopentanolmischungen kann bekanntermaßen durch Umsetzung und Analysieren der gebildeten Ester erfolgen.

Die Veresterung findet vorzugsweise in Anwesenheit eines Katalysators statt. Grundsätzlich können dazu bekannte und für die Veresterung geeignete Katalysatorsysteme eingesetzt werden. Geeignete Katalysatoren für die Veresterung zur Herstellung der erfindungsgemäßen Tetraisopentylester der 1,2,3,4-Butantetracarbonsäure sind Titanat-Katalysatoren, beispielsweise Tetra-n-Butyltitanat, Zirkonate oder Sulfonsäuren.

Die Veresterung zur Herstellung der erfindungsgemäßen Ester erfolgt bevorzugt bei einer Temperatur von 120 bis 250 °C, weiterhin bevorzugt bei einer Temperatur von 140 bis 230 °C, besonders bevorzugt bei einer Temperatur von 160 bis 215 °C. Der Druck bei der Veresterung sollte vorzugsweise nicht zu hoch sein, da dies die Siedetemperatur und dadurch auch die Veresterungstemperatur erhöhen würde. Der Druck bei der Veresterung liegt deshalb bei 3 bar absolut oder weniger, besonders bevorzugt bei nicht weniger als 0,5 bar absolut.

Während einer Veresterung entsteht durch die Reaktion der Säuregruppe mit einem Alkohol Wasser. Dieses Wasser wird auch als Reaktionswasser bezeichnet. In einer bevorzugten Ausführungsform der vorliegenden Erfindung wird zumindest ein Teil des entstehenden Reaktionswasser während der noch laufenden Reaktion abgetrennt. Dadurch kann u. a. das Gleichgewicht der Reaktion in die richtige Richtung verschoben werden.

Der Fortgang der Veresterungsreaktion kann durch die Überwachung eines Parameters überwacht werden. Möglich ist beispielsweise die Überwachung der Säurezahl oder der Wassermenge. Möglich ist auch die Überwachung mittels Gaschromatographie, wo der Anteil von Edukten und/oder Produkten ermittelt werden kann.

Ist die Reaktion ausreichend weit fortgeschritten kann die Reaktion beendet werden. Dies kann beispielsweise über die Zugabe einer Base, beispielsweise Natronlauge geschehen. Der Katalysator wird dabei in der Regel zerstört. Entsprechendes ist dem Fachmann wohlbekannt. Anschließend kann die Reaktionslösung mit bekannten Verfahren aufgebarbeitet werden, beispielsweise mittels thermischer Trennung.

Die erfindungsgemäße Mischung von Tetraisopentylestern der 1,2,3,4-Butantetracarbonsäure können auch durch Umesterung des Tetramethylesters der 1,2,3,4-Butantetracarbonsäure mit einer geeigneten Isopentanolmischung hergestellt werden. Isopentanolmischung ist in diesem Zusammenhang so zu verstehen, dass diese Mischung zumindest 2-Methylbutanol und n-Pentanol und/oder 3-Methylbutanol enthält. Bei der Veresterung sollte verständlicherweise darauf geachtet werden, dass die Mischung ausreichend 2-Methylbutanol enthält, um die beanspruchte Menge an 2-Methylbutylresten im gebildeten Ester zu erhalten. Die Auswahl geeigneter Isopentanolmischungen kann bekanntermaßen durch Umsetzung und Analysieren der gebildeten Ester erfolgen. Der Fortgang der Umesterungsreaktion kann durch die Überwachung eines Parameters überwacht werden. Möglich ist hier nur die Überwachung mittels Gaschromatographie, wo der Anteil von Edukten und/oder Produkten ermittelt werden kann.

Die erfindungsgemäße Mischung von Tetraisopentylestern der 1,2,3,4-Butantetracarbonsäure weist vorteilhafte Eigenschaften beim Einsatz als Weichmacher für Polymere auf. Ein weiterer Gegenstand der vorliegenden Erfindung ist deshalb die Verwendung der Mischung von Tetraisopentylestern der 1,2,3,4-Butantetracarbonsäure als Weichmacher für Polymere. Geeignete Polymere sind unten aufgeführt, bevorzugt sind jedoch PVC oder Vinylchlorid-enthaltende Copolymere.

Gegenstand der vorliegenden Erfindung ist auch eine Weichmacherzusammensetzung, die neben der Mischung von Tetraisopentylestern der 1,2,3,4-Butantetracarbonsäure einen weiteren Weichmacher enthält. Je nach Anwendungszweck können ein oder mehrere zusätzliche, insbesondere von der erfindungsgemäßen Mischung von Tetraisopentylestern der 1,2,3,4-Butantetracarbonsäure verschiedene, Weichmacher in der Weichmacherzusammensetzung enthalten sein, um die Eigenschaften der resultierenden Weichmacherzusammensetzung gezielt einzustellen. Nach einer besonders bevorzugten Ausführungsform umfasst die Weichmacherzusammensetzung jedoch weniger als 5 Gew.-%, weiterhin bevorzugt weniger als 0,5 Gew.-%, besonders bevorzugt weniger als 0,1 Gew.-% Phthalate.

Der zusätzliche Weichmacher in der erfindungsgemäßen Weichmacherzusammensetzung kann aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, Cyclohexandicarboxylaten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten, Isophthalaten, Trimellitaten und oligomeren oder polymeren Estern auf Basis von Adipin-, Bernstein- oder Sebacinsäure, ausgewählt werden. In einer bevorzugten Ausführungsform der vorliegenden Erfindung enthält die Weichmacherzusammensetzung einen weiteren Weichmacher, der aus der Gruppe, bestehend aus Alkylbenzoaten, Alkylsulfonsäureestern des Phenols, Dialkyladipaten, Glycerinestern, C4-C6-Säuren von Polyolen, Citronensäuretrialkylestern, acetylierten Citronensäuretrialkylestern, Glykoldibenzoaten, Trialkylestern der Trimellitsäure, Dialkylterephthalaten, Dialkylphthalaten, Dialkylisophthalaten, Estern der Furandicarbonsäure, Dialkanoylestern von Dianhydrohexitolen (z.B. Isosorbid), epoxidierten Fettsäurealkylestern, Polymerweichmachern, beispielsweise der Polyadipate, und Dialkylestern der 1,2-, 1,3- oder 1,4-Cyclohexandicarbonsäure, ausgewählt wird.

In einer weiterhin bevorzugten Ausführungsform wird der weitere Weichmacher, der in der Weichmacherzusammensetzung enthalten ist, aus der Gruppe, bestehend aus C8- bis C13-Alkylbenzoaten, C4- bis C10-Dialkyladipaten, Pentaerythrit-tetravalerat, acetylierten Citronensäuretrialkylestern mit C4 bis C9-Alkylgruppen, C4- bis C10-Trialkyltrimellitaten, C4- bis C9-Dialkylterephthalaten, C4- bis C13-Dialkylphthalaten, insbesondere C9- bis C13-Dialkylphthalaten und C4- bis C10-Dialkylestern der 1,2-, 1,3- oder 1,4-Cyclohexandicarbonsäure, ausgewählt.

Gegenstand der vorliegenden Erfindung ist daher auch eine Kunststoffzusammensetzung, enthaltend die erfindungsgemäße Mischung von Tetraisopentylestern der 1,2,3,4-Butantetracarbonsäure oder die Weichmacherzusammensetzung und ein oder mehrere Polymere.

Geeignete Polymere sind vorzugsweise ausgewählt aus der Gruppe, die gebildet wird durch PVC, Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA),

Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikone.

In einer bevorzugten Ausführungsform ist mindestens ein Polymer bzw. sind vorzugsweise mindestens 90 Gew.-% der mehreren Polymere in der Weichmacherzusammensetzung ausgewählt aus der Gruppe bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat. Besonders bevorzugt ist hiervon PVC.

Die Menge an der erfindungsgemäßen Mischung von Tetraisopentylestern der 1,2,3,4-Butantetracarbonsäure oder der Weichmacherzusammensetzung in der Kunststoffzusammensetzung beträgt vorzugsweise 5 bis 150 Massenteile, bevorzugt 10 bis 120 Massenteile, besonders bevorzugt 15 bis 110 Massenteile und ganz besonders bevorzugt 20 bis 100 Massenteile pro 100 Massenteile Polymer. Es sind aber auch ein oder mehrere Polymere enthaltende Zusammensetzungen denkbar, die weniger als 20 Massenteile der erfindungsgemäßen Tetraisopentylester der 1,2,3,4-Butantetracarbonsäure pro 100 Massenteile Polymer umfassen.

Nachfolgend sind spezielle Zusammensetzungen bzw. Mischungen von Weichmachern mit den erfindungsgemäßen Mischungen von Tetraisopentylestern der 1,2,3,4-Butantetracarbonsäure angegeben, die im Rahmen der vorliegenden Erfindung bevorzugt sind.

Ein bevorzugter Gegenstand der vorliegenden Erfindung ist eine Kunststoffzusammensetzung, die die Mischung von Tetraisopentylestern der 1,2,3,4-Butantetracarbonsäure und Diethylhexylterephthalat (DEHT oder DOTP) und mindestens ein Polymer, vorzugsweise PVC umfasst.

Ein weiterer bevorzugter Gegenstand der vorliegenden Erfindung ist eine Kunststoffzusammensetzung, die die Mischung von Tetraisopentylestern der 1,2,3,4-Butantetracarbonsäure und 1,2- oder 1,4-Cyclohexandicarbonsäureester, insbesondere die entsprechenden Diisononyl- oder Di-2-ethylhexylester, und mindestens ein Polymer, vorzugsweise PVC umfasst.

Ebenfalls bevorzugt ist eine Kunststoffzusammensetzung, die die Mischung von Tetraisopentylestern der 1,2,3,4-Butantetracarbonsäure und Trialkylester der Trimellitsäure, in denen die Alkylreste 4 und mehr Kohlenstoffatome, vorzugsweise 5, 8, 9 oder 10 Kohlenstoffatome aufweisen, und mindestens ein Polymer, vorzugsweise PVC umfasst.

Ebenfalls bevorzugt ist eine Kunststoffzusammensetzung, die die Mischung von Tetraisopentylestern der 1,2,3,4-Butantetracarbonsäure und Trialkylester der 1,24-Cyclohexantricarbonsäure, in denen die Alkylreste 4 und mehr Kohlenstoffatome, vorzugsweise 5, 8, 9 oder 10 Kohlenstoffatome aufweisen, und mindestens ein Polymer, vorzugsweise PVC umfasst.

Ein weiterer bevorzugter Gegenstand der vorliegenden Erfindung ist eine Kunststoffzusammensetzung, die die Mischung von Tetraisopentylestern der 1,2,3,4-Butantetracarbonsäure und einen schnell gelierenden Weichmacher ausgewählt aus der Gruppe, bestehend aus Dibutylterephthalat, Di(iso)-pentylterephthalat, Isodecylbenzoat, Isononylbenzoat, Acetyl-tributylcitrat, Tributylcitrat, Dipropylenglycoldibenzoat, Diethylenglycoldibenzoat, Triethylenglycoldibenzoat und Mischungen von zwei oder mehr davon, und mindestens ein Polymer, vorzugsweise PVC enthält.

Die erfindungsgemäße Kunststoffzusammensetzung ist vorzugsweise Bestandteil eines Klebstoffs, einer Dichtungsmasse, einer Beschichtungsmasse, eines Lacks, einer Farbe, eines Plastisols, eines Dryblends, eines Schaums, eines Kunstleders, eines Fußbodenbelags, insbesondere dessen Deck- oder Schaumschicht, einer Dachbahn, eines Unterbodenschutzes, einer Gewebebeschichtung, eines Kabels, einer Drahtisolierung, eines Schlauchs, eines Extrusionsartikels, einer Folie, eines Gegenstands im Automobilinnenbereich, einer Tapete, einer Tinte, eines Spielzeugs, einer Kontaktfolie, einer Lebensmittelverpackung oder eines medizinischen Artikels, insbesondere eines Schlauchs oder eines Blutbeutels.

Gegenstand der vorliegenden Erfindung ist deshalb auch die Verwendung der Kunststoffzusammensetzung in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

Es wurde bereits erwähnt, dass die Begriffe isopentyl", "Iso-Pentylreste" oder ähnliches als Synonym zu dem Begriff "Pentyl" zu verstehen sind und nicht auf eine einzige spezielle C5-Alkylgruppe hinweisen. Insofern könnten die einzelnen erfindungsgemäßen Gegenstände der vorliegenden Erfindung auch wie folgt beschrieben werden:

1. Mischung von Tetrapentylestern der 1,2,3,4-Butantetracarbonsäure, wobei 45 bis 60 Mol% der iso-Pentylreste in der Mischung 2-Methylbutylreste sind.

2. Mischung nach Gegenstand 1, wobei 50 bis 60 Mol% der der Pentylreste in der Mischung 2-Methylbutylreste sind.

3. Mischung nach Gegenstand 1, wobei 40 bis 55 Mol% der Pentylreste in der Mischung n-Pentylreste, 3-Methylbutylreste oder eine Mischung aus n-Pentylresten und 3-Methylbutylresten sind.

4. Mischung nach Gegenstand 2, wobei 40 bis 50 Mol% der Pentylreste in der Mischung n-Pentylreste, 3-Methylbutylreste oder eine Mischung aus n-Pentylresten und 3-Methylbutylresten sind.

5. Verfahren zur Herstellung der Mischung von Tetrapentylestern der 1,2,3,4-Butantetracarbonsäure nach einem der Gegenstände 1 bis 4, wobei die Herstellung entweder mittels Veresterung von 1,2,3,4-Butantetracarbonsäure mit einer Pentanolmischung oder mittels Umesterung des Tetramethylesters der 1,2,3,4-Butantetracarbonsäure mit einer Pentanolmischung erfolgt.

6. Verfahren nach Gegenstand 5, wobei das bei der Veresterung gebildete Reaktionswasser während der Reaktion abgetrennt wird.

7. Verfahren nach Gegenstand 5 oder 6, wobei die Veresterung bei einer Temperatur im Bereich von 120 bis 250 °C durchgeführt wird.

8. Verwendung der Mischung von Tetrapentylestern der 1,2,3,4-Butantetracarbonsäure nach einem der Gegenstände 1 bis 4 als Weichmacher für Polymere, insbesondere für PVC oder Vinylchlorid-enthaltende Copolymere.

9. Weichmacherzusammensetzung, die die Mischung von Tetrapentylestern der 1,2,3,4-Butantetracarbonsäure nach einem der Gegenstände 1 bis 4 und mindestens eine weitere weichmachende Verbindung enthält.

10. Weichmacherzusammensetzung nach Gegenstand 9, wobei die weitere weichmachende Verbindung aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, Cyclohexandicarboxylaten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten, Trimellitaten und oligomeren oder polymeren Estern auf Basis von Adipin-, Bernstein- oder Sebacinsäure, ausgewählt wird.

11. Weichmacherzusammensetzung nach Gegenstand 9 oder 10, wobei die Weichmacherzusammensetzung weniger als 5 Gew.-%, weiterhin bevorzugt weniger als 0,5 Gew.- %, besonders bevorzugt weniger als 0,1 Gew.-% an Phthalaten enthält.

12. Kunststoffzusammensetzung, enthaltend die die Mischung von Tetrapentylestern der 1,2,3,4-Butantetracarbonsäure nach einem der Gegenstände 1 bis 4 oder die Weichmacherzusammensetzung nach einem der Gegenstände 9 bis 11 und ein oder mehrere Polymere.

13. Kunststoffzusammensetzung nach Gegenstand 12, wobei der oder die Polymere aus der Gruppe, bestehend aus PVC, Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), CelluloseAcetat/Butyrat (CAB), Gummi und Silikone ausgewählt

wird.

14. Kunststoffzusammensetzung nach Gegenstand 12 oder 13, wobei die Menge an der erfindungsgemäßen Mischung von Tetrapentylestern der 1,2,3,4-Butantetracarbonsäure oder der Weichmacherzusammensetzung in der Kunststoffzusammensetzung 5 bis 150 Massenteile, bevorzugt 10 bis 120 Massenteile und besonders bevorzugt 15 bis 110 Massenteile pro 100 Massenteile Polymer beträgt.

15. Verwendung der Kunststoffzusammensetzung nach einem der Gegenstände 12 bis 14 in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln eingesetzt wird.

Die Erfindung wird nachfolgend anhand von Beispielen erläutert. Die Beispiele sollen einen Einblick geben und sind nicht einschränkend zu verstehen.

Beispiele

Synthese der Mischung von Tetraisopentylestern der 1,2,3,4-Butantetracarbonsäure

In eine Apparatur wurden 5 mol 1,2,3,4-Butantetracarbonsäure (Alfa Aesar, Reinheit: > 98 %) und entsprechende Mengen an den Alkoholen 2-Methylbutanol, n-Pentanol und 3-Methylbutanol eingefüllt (2-Methylbutanol: Firma Sigma Aldrich, Reinheit ≥ 99 %; 3-Methylbutanol: Firma Honeywell, Reinheit ≥ 98,5 %; n-Pentanol: Firma Honeywell, Reinheit ≥ 99 %). Dazu wurden 3,4 g Tetra-n-Butyltitanat (0,01 Mol, Sigma Aldrich, Reinheit: > 97 %) als Katalysator hinzugegeben und die Reaktion gestartet. Die Reaktion verlief unter Einperlung von Stickstoff. Die Reaktanden wurden langsam bis zu einer Reaktionstemperatur von 200 °C erhitzt. Nach Erreichen der Reaktionstemperatur wurden zusätzlicher Alkohol zudosiert. Bei der Dosierung wurde darauf geachtet, dass die Reaktionstemperatur nicht unter 200°C fiel.

Im Verlauf der Veresterung fielen 360 ml Wasser an (20 mol Reaktionswasser). Das bei der Reaktion entstehende Wasser wurde kontinuierlich über einen Wasserauskreiser entfernt. Nach Erreichen von 360 ml Reaktionswasser wurde die Säurezahl (SZ) anhand einer Probe aus der Reaktion bestimmt. In regelmäßigen Abständen wurde der Reaktionsfortschritt über die SZ kontrolliert bis eine SZ von < 0,5 mg KOH pro g Probe erreicht wurde. Anschließend wurde der Ansatz abgekühlt und der Katalysator durch Zugabe von Natronlauge zerstört.

Der Reaktionsaustrag aus der Veresterung wurde in einen Rührkolben mit Rührer umgefüllt. Die Apparatur wurde mit Stickstoff (6 l/h) gespült. Unter Vakuum (ca. 1 - 5 mbar) wurde der Reaktionsaustrag langsam aufgeheizt und die Temperatur bis auf 160°C erhöht. Nach vollständiger Destillation des Überschussalkohols wurde das Produkt auf 80 °C abgekühlt und filtriert. Das Filtrat wurde mittels einer NMR-Analyse untersucht, um die Zusammensetzung der Esterreste im Produkt zu ermitteln.

NMR-Analyse

Die Ermittlung der Zusammensetzung der Tetra-isopentyl-1,2,3,4-butantetracarbonsäureester, das heißt der jeweilige Anteil der unterschiedlichen isomeren Butylreste an der Gesamtheit aller Butylreste, kann beispielsweise durch $^{1}$H-NMR und $^{13}$C-NMR Spektroskopie erfolgen. Aufgrund der höheren Genauigkeit wurde die Ermittlung der Zusammensetzung hier mit Hilfe der $^{1}$H-NMR-Spektroskopie an einer Lösung der Tetra-isopentyl-1,2,3,4-butantetracarbonsäureestergemische in Deuterochloroform ($CDCl_3$) durchgeführt. Für die Aufnahme der 1H-NMR-Spektren wurden ca. 20 mg Substanz in ca. 0,6 ml $CDCl_3$ (enthalten jeweils ca. 1 Massen-% TMS) gelöst und in ein NMR-Röhrchen mit einem Durchmesser von 5 mm gefüllt. Das verwendete $CDCl_3$ wurden zunächst über Molekularsieb getrocknet, um Verfälschungen der Messwerte durch eventuell vorhandenes Wasser auszuschließen.

Die NMR-spektroskopischen Untersuchungen können prinzipiell mit jedem handelsüblichen NMR-Gerät durchgeführt werden. Für die vorliegenden NMR-spektroskopischen Untersuchungen wurde ein Gerät vom Typ Avance 500 der Firma Bruker eingesetzt. Die $^{1}$H-NMR-Spektren wurden bei einer Temperatur von 300 K mit einem Delay (Verzögerung) von d1 = 5 Sekunden, 32 Scans (Durchgänge), einer Pulslänge von ca. 12 $\mu$s (30° Anregungspuls) und einer Sweep Width (Spektrale Breite) von 10000 Hz mit einem 5 mm Prodigy Cryo-Probenkopf (CPPBO) mit Bruker Cooling Unit (BCU 05) aufgenommen. Die Resonanzsigna-le wurden gegen die chemischen Verschiebungen von Tetramethylsilan (TMS = 0 ppm) als internen Standard aufgezeichnet. Mit anderen handelsüblichen NMR-Geräten werden mit den gleichen Betriebsparametern vergleichbare Ergebnisse erhalten.

Die folgende Abbildung 1 soll lediglich der Nachvollziehbarkeit der erläuterten Bestimmungsmethode dienen. Relevant hierbei ist die zugewiesene Nummerierung der Kohlenstoffatome der Methylgruppen sowie der Methylengruppen benachbart zum Sauerstoffatom jeweils in einem n-Pentylrest ($C14H_3$ und C10Hz), in einem 2-Methylbutylrest ($C23H_3$, $C24H_3$ und $C20H_2$) und in einem 3-Methylbutylrest ($C33H_3$ und $C30H_2$).

Abbildung 1: Nummerierung der Kohlenstoffatome in den unterschiedlichen Pentylresten

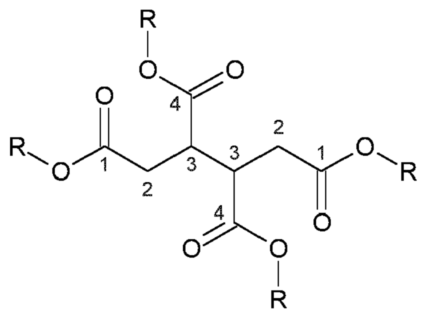

n-Pentanol

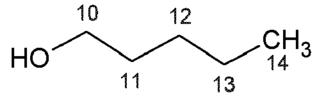

2-Methylbutanol

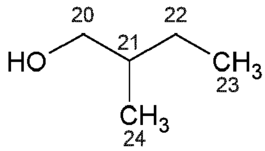

3-Methylbutanol

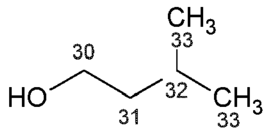

wobei jedes R = n-Pentanol, 2-Methylbutanol oder 3-Methylbutanol ist.

Die erhaltenen $^1$H-NMR-Spektren der Gemische von Tetra-isopentyl-1,2,3,4-butantetracarbonsäureester weisen im Bereich zwischen 0,8 ppm bis 1,0 ppm Resonanzsignale auf, die durch die Signale der Wasserstoffatome der Methylgruppe(n) der isomeren Pentylsubstituenten gebildet werden ($C14H_3$, $C23H_3$, $C24H_3$, $C33H_3$). Die Signale im Bereich der chemischen Verschiebungen von 3,60 bis 4,40 ppm können im Wesentlichen den Wasserstoffatomen der Methylengruppe, welche dem Sauerstoff des Alkoholrests benachbart ist, zugeordnet werden ($C10H_2$, $C20H_2$, $C30H_2$). Hierbei erfahren die Protonen an C20 aufgrund des benachbarten tertiären Kohlenstoffatoms eine Hochfeldverschiebung und erscheinen zwischen 4,03 ppm bis 3,84 ppm, während die Protonen an C10 und C30 Signale bei tieferen Verschiebungen von 4,20 ppm bis 4,03 ppm liefern und sich wechselseitig überlagern.

Die Quantifizierung erfolgte durch vergleichende Bestimmung der Fläche unter den jeweiligen Resonanzsignalen, d. h. der vom Signal von der Grundlinie eingeschlossenen Fläche. Handelsübliche NMR-Software verfügt über Programmfunktionen zur Integration der Signalfläche. In der vorliegenden NMR-spektroskopischen Untersuchung wurde die Integration mit Hilfe der Software TopSpin®, Version 3.6 durchgeführt.

Zur Bestimmung des mittleren Verzweigungsgrades der isomeren Pentylreste im erfindungsgemäßen Gemisch wird zunächst der Integralwert der Signale im Bereich von 0,68 bis 1,11 ppm ($I(CH_3)$) durch den Integralwert der Signale im Bereich von 3,60 bis 4,40 ppm ($I(OCH_2)$) dividiert. Auf diese Weise erhält man ein Intensitätsverhältnis, welches das Verhältnis der Anzahl der Wasserstoffatome, die in einer Methylgruppe vorhanden sind, zur Anzahl der Wasserstoffatome, die in einer einem Sauerstoff benachbarten Methylengruppe vorhanden sind, angibt. Da pro Methylgruppe drei Wasserstoffatome und in jeder einem Sauerstoff benachbarten Methylengruppe zwei Wasserstoffatome vorhanden sind, müssen die Intensitäten jeweils durch 3 bzw. 2 geteilt werden, um das Verhältnis der Anzahl der Methylgruppen zur Anzahl der einem Sauerstoff benachbarten Methylengruppe im Pentylrest zu erhalten. Da ein linearer n-Pentylrest, welcher nur eine Methylgruppe und eine einem Sauerstoff benachbarte Methylengruppe aufweist, keine Verzweigung enthält und demnach einen Verzweigungsgrad von 0 aufweisen muss, muss von dem Verhältnis noch der Betrag 1 subtrahiert werden.

Der mittlere Verzweigungsgrad V kann also gemäß der Formel

$$V = 2/3 * I(CH_3)/I(OCH_2) - 1$$

aus dem gemessenen Intensitätsverhältnis berechnet werden. Hierin bedeutet V mittlerer Verzweigungsgrad, $I(CH_3)$ Flächenintegral, welches den Methylwasserstoffatomen zugeordnet ist, und I(OCHz) Flächenintegral der Methylenwasserstoffatome in Nachbarschaft zum Sauerstoff.

Im Produkt können 2-Methylbutylreste und 3-Methylbutylreste, welche jeweils einen Verzweigungsgrad von 1 aufweisen, sowie n-Pentylreste, welche einen Verzweigungsgrad von 0 besitzen, enthalten sein, womit der mittlere Verzweigungsgrad eines Tetraisopentylesters stets bei maximal 1 liegt. Aus der Abweichung des mittleren Verzweigungsrades von dem Wert 1 kann daher der Stoffmengenanteil an *n*-Pentylresten ($x_{Pentyl}$) im Molekül ermittelt werden.

$$x_{Pentyl} = 1 - V$$

Der Anteil an 2-Methylbutylresten kann nicht mit Hilfe der Integration der Basislinien-getrennten Signale im Bereich von 3,60 bis 4,40 ppm berechnet werden.

Die Trennung der Signale der Protonen an C20 ($C^{20}H_2$; Multiplett zwischen 3,95 und 4,28 ppm) von den Signalen der Protonen an C10 und C30 ($C^{10}H_2$ und $C^{30}H_2$; Multiplett zwischen 4,29 und 4,55 ppm) erfolgt auch hier im Minimum des Taleinschnittes zwischen den Signalgruppen.

Der Stoffmengenanteil an 2-Methylbutylresten ($x_{2\text{-Methylbutyl}}$) lässt sich gemäß der Formel

$$x_{2\text{-Methylbutyl}} = I(OC^{19}H_2) / I(OCH_2)$$

durch das In-Verhältnis-Setzen der Intensität der Signale für die $OC^{20}H_2$-Protonen ($I(OC^{20}H_2)$) zu der Intensität aller $OCH_2$-Protonen ($I(OCH_2)$) berechnen.

Der Stoffmengenanteil an 3-Methylbutylresten ($x_{3\text{-Methylbutyl}}$) ergibt sich somit aus der Differenz der beiden vorherigen Stoffmengenanteile zu 1.

$$x_{3\text{-Methylbutyl}} = 1 - x_{2\text{-Methylbutyl}} - x_{Pentyl}$$

Mittels der vorgenannten Methode konnten die Anteile der jeweiligen Reste in den Proben bestimmt werden (s. Tabelle 1):

Tabelle 1: Zusammensetzung der Estergemische gemäß NMR-Analyse

| | Anteil 2-Methylbutyl (Produkt) [%] | Anteil 3-Methylbutyl (Produkt) [%] | Anteil n-Pentyl (Produkt) [%] |
|---|---|---|---|
| Bsp. 1* | 52,2 | 0 | 47,8 |
| Bsp. 2* | 59,5 | 0 | 40,5 |
| Bsp. 3* | 59,0 | 21,2 | 19,8 |
| Bsp. 4* | 45,3 | 0 | 54,7 |
| Vgl. Bsp. 5 | 39,2 | 0 | 60,8 |
| Vgl. Bsp. 6 | 0 | 0 | 100 |
| *Erfindungsgemäß | | | |

Die Estergemische aus Tabelle 2 wurden auf anwendungsrelevante Eigenschaften hin untersucht.

Messung Eindickverhalten

Es wurden PVC-Plastisole hergestellt, wie sie beispielsweise zur Fertigung von Deckstrichfilmen verwendet werden. Die Mengen in den Plastisolrezepturen sind jeweils in Massenanteilen (phr) angegeben. Die Rezeptur ist in Tabelle 2 dargestellt.

Tabelle 2: Plastisolrezeptur

| | phr |
|---|---|
| PVC (Vestolit P1430 K70 - Ultra; Fa. Vestolit) | 100 |
| Weichmacher oder Weichmachergemisch | 50 |
| epoxidiertes Sojabohnenöl als Co-Stabilisator (Edenol D81, Fa. Emery Oleochemicals) | 3 |
| Thermostabilisator auf Ba/Zn-Basis ((Reagens MBL 197/9PF, Fa. Reagens) | 2 |

Zuerst wurden die flüssigen und dann die pulverförmigen Bestandteile in einen PE-Becher eingewogen. Von Hand wurde die Mischung mit einem Spatel so eingerührt, dass kein unbenetztes Pulver mehr vorhanden war. Der Mischbecher wurde dann in die Klemmvorrichtung eines Dissolverrührers eingespannt. Nach Einschalten des Rührers wurde die Drehzahl langsam auf ca. 2000 UpM (Umdrehungen pro Minute) erhöht. Währenddessen wurde das Plastisol vorsichtig entlüftet. Dazu wurde der Druck auf einen Druck unterhalb 20 mbar eingestellt. Sobald das Plastisol eine Temperatur von ca. 30°C erreicht hatte, wurde die Drehzahl auf ca. 350 UpM gesenkt. Ab jetzt wurde das Plastisol für 9 Minuten bei dieser Drehzahl und einem Druck unterhalb von 20 mbar entlüftet. Damit wurde sichergestellt, dass es bei der Homogenisierung des Plastisols nicht zu einem vorzeitigen Angelieren kommen konnte.

Die Messung des Eindickverhaltens ist grundsätzliche die Messung der Viskosität der hergestellten Plastisole zu verschiedenen Zeitpunkten geteilt durch die Viskosität kurz nach Ansatz. Die Viskosität der Plastisole wurde mit einem Rheometer Physica MCR 101 (Firma Anton Paar Germany GmbH) mit Hilfe der zugehörigen Software durchgeführt, wobei der Rotationsmodus und das Messsystem CC27 verwendet wurden. Die Messung wurde durchgeführt, nachdem die Plastisole seit ihrer Herstellung 24 Stunden bei 25 °C temperiert wurden.

Während der Messung wurden folgende Punkte angesteuert:

- Vorscherung von 100 $s^{-1}$ für einen Zeitraum von 60 Sekunden, bei der keine Messwerte aufgenommen wurden
- Scherraten-Abwärtsrampe von 200 $s^{-1}$ bis 0,1 $s^{-1}$. Es wurden 30 Messpunkte aufgenommen mit einer Messpunktdauer von je 10 Sekunden.

Die Messungen wurden bei Raumtemperatur nach 2 Stunden, 24 Stunden und 7 Tagen (jeweils bezogen auf den Zeitpunkt der Herstellung der Plastisole) durchgeführt. Bestimmt wurde jeweils die Viskosität, die bei einer Scherrate von 0,1 $s^{-1}$, 1 $s^{-1}$ und 10 $s^{-1}$ erhalten wurde. Die Ergebnisse sind in Tabelle 3 gezeigt.

Der prozentuale Anstieg der Viskosität über die Zeit für die einzelnen Scherraten wurde wie folgt bestimmt:

1 d = (Viskosität nach 24 Stunden - Viskosität nach 2 Stunden) / Viskosität nach 2 Stunden
7 d = (Viskosität nach 7 Tagen - Viskosität nach 2 Stunden) / Viskosität nach 2 Stunden

Tabelle 3: Eindickverhalten der Proben

| Eindickverhalten der Tetraisopentylester der Butantetracarbonsäuren bei verschiedenen Scherraten | | | | | | |
|---|---|---|---|---|---|---|
| Scherrate | 0,1 $s^{-1}$ | | 1 $s^{-1}$ | | 10 $s^{-1}$ | |
| Messzeitpunkt | 1 d | 7 d | 1 d | 7 d | 1 d | 7 d |
| Beispiel 1* (52 Mol% 2-MB) | 16,8 | 55.8 | 15.2 | 48.1 | 10.0 | 31.4 |
| Beispiel 2* (59 Mol% 2-MB) | 18,7 | 60.9 | 15.7 | 49.3 | 9.7 | 30.1 |
| Beispiel 3* (59 Mol% 2-MB, 21% Pe, 19% 3-Mb) | 16,0 | 55.6 | 14.8 | 45.8 | 8.1 | 24.0 |
| Beispiel 4 (45 Mol% 2-MB)* | 15,8 | 54.1 | 13.4 | 45.5 | 9.1 | 30.4 |
| Vergleichsbeispiel 5 (39 Mol% 2-MB) | 19,4 | 63.5 | 16.5 | 51.7 | 10.1 | 32.8 |
| Vergleichsbeispiel 6 (reines n-Pe) | 18,3 | 63.5 | 16.2 | 54.7 | 12.0 | 39.3 |

Zusammengefasst kann man erkennen, dass das Eindickverhalten im beanspruchten Bereich besser ist als außerhalb des beanspruchten Bereichs. Der Anstieg der Viskosität gegenüber der Zeit ist im beanspruchten Bereich geringer als bei den Vergleichsbeispielen.

## Patentansprüche

1. Mischung von Tetraisopentylestern der 1,2,3,4-Butantetracarbonsäure, wobei 45 bis 60 Mol% der iso-Pentylreste in der Mischung 2-Methylbutylreste sind.

2. Mischung nach Anspruch 1, wobei 50 bis 60 Mol% der der iso-Pentylreste in der Mischung 2-Methylbutylreste sind.

3. Mischung nach Anspruch 1, wobei 40 bis 55 Mol% der iso-Pentylreste in der Mischung n-Pentylreste, 3-Methylbutylreste oder eine Mischung aus n-Pentylresten und 3-Methylbutylresten sind.

4. Mischung nach Anspruch 2, wobei 40 bis 50 Mol% der iso-Pentylreste in der Mischung n-Pentylreste, 3-Methylbutylreste oder eine Mischung aus n-Pentylresten und 3-Methylbutylresten sind.

5. Verfahren zur Herstellung der Mischung von Tetraisopentylestern der 1,2,3,4-Butantetracarbonsäure nach einem der Ansprüche 1 bis 4, wobei die Herstellung entweder mittels Veresterung von 1,2,3,4-Butantetracarbonsäure mit einer Isopentanolmischung oder mittels Umesterung des Tetramethylesters der 1,2,3,4-Butantetracarbonsäure mit einer Isopentanolmischung erfolgt.

6. Verfahren nach Anspruch 5, wobei das bei der Veresterung gebildete Reaktionswasser während der Reaktion abgetrennt wird.

7. Verfahren nach Anspruch 5 oder 6, wobei die Veresterung bei einer Temperatur im Bereich von 120 bis 250 °C durchgeführt wird.

8. Verwendung der Mischung von Tetraisopentylestern der 1,2,3,4-Butantetracarbonsäure nach einem der Ansprüche 1 bis 4 als Weichmacher für Polymere, insbesondere für PVC oder Vinylchlorid-enthaltende Copolymere.

9. Weichmacherzusammensetzung, die die Mischung von Tetraisopentylestern der 1,2,3,4-Butantetracarbonsäure nach einem der Ansprüche 1 bis 4 und mindestens eine weitere weichmachende Verbindung enthält.

10. Weichmacherzusammensetzung nach Anspruch 9, wobei die weitere weichmachende Verbindung aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, Cyclohexandicarboxylaten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten, Trimellitaten und oligomeren oder polymeren Estern auf Basis von Adipin-, Bernstein- oder Sebacinsäure, ausgewählt wird.

11. Weichmacherzusammensetzung nach Anspruch 9 oder 10, wobei die Weichmacherzusammensetzung weniger als 5 Gew.-%, weiterhin bevorzugt weniger als 0,5 Gew.-%, besonders bevorzugt weniger als 0,1 Gew.-% an Phthalaten enthält.

12. Kunststoffzusammensetzung, enthaltend die die Mischung von Tetraisopentylestern der 1 ,2,3,4-Butantetracarbonsäure nach einem der Ansprüche 1 bis 4 oder die Weichmacherzusammensetzung nach einem der Ansprüche 9 bis 11 und ein oder mehrere Polymere.

13. Kunststoffzusammensetzung nach Anspruch 12, wobei der oder die Polymere aus der Gruppe, bestehend aus PVC, Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB),

Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikone ausgewählt wird.

**14.** Kunststoffzusammensetzung nach Anspruch 12 oder 13, wobei die Menge an der erfindungsgemäßen Mischung von Tetraisopentylestern der 1,2,3,4-Butantetracarbonsäure oder der Weichmacherzusammensetzung in der Kunststoffzusammensetzung 5 bis 150 Massenteile, bevorzugt 10 bis 120 Massenteile und besonders bevorzugt 15 bis 110 Massenteile pro 100 Massenteile Polymer beträgt.

**15.** Verwendung der Kunststoffzusammensetzung nach einem der Ansprüche 12 bis 14 in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln eingesetzt wird.

Europäisches Patentamt
European Patent Office
Office européen des brevets

# EUROPÄISCHER RECHERCHENBERICHT

Nummer der Anmeldung
EP 23 19 5912

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | US 2011/257317 A1 (BAUGH LISA SAUNDERS [US] ET AL) 20. Oktober 2011 (2011-10-20)<br>* Zusammenfassung; Ansprüche 1,2,7,43,47,53,54 *<br>* Beispiele *<br>----- | 1-15 | INV.<br>C08K5/00<br>C08K5/11<br>C08K5/1515<br>C07C67/02<br>C07C67/03<br>C07C67/08<br>C07C69/34 |
| A | DATABASE CA [Online]<br>CHEMICAL ABSTRACTS SERVICE, COLUMBUS, OHIO, US;<br>15. September 1984 (1984-09-15),<br>"Poly(vinyl chloride) resin compositions",<br>XP002808623,<br>gefunden im STN<br>Database accession no. 1984:492123<br>* Zusammenfassung *<br>-& JP S59 22950 A (CHISSO CORP)<br>6. Februar 1984 (1984-02-06)<br>* Zusammenfassung; Anspruch 1 *<br>* Weichmacher A und B *<br>----- | 1-15 | |
| A | DATABASE CA [Online]<br>CHEMICAL ABSTRACTS SERVICE, COLUMBUS, OHIO, US;<br>5. Februar 1994 (1994-02-05),<br>FUJITANI, YOSHIFUMI ET AL:<br>"Halogen-containing polymer compositions with high usable temperature",<br>XP002808624,<br>gefunden im STN<br>Database accession no. 1994:55944<br>* Zusammenfassung *<br>-& JP H05 98106 A (NEW JAPAN CHEM CO LTD)<br>20. April 1993 (1993-04-20)<br>* Zusammenfassung *<br>* Beispiele *<br>----- | 1-15 | RECHERCHIERTE SACHGEBIETE (IPC)<br>C08K<br>C08L<br>C07C |

Der vorliegende Recherchenbericht wurde für alle Patentansprüche erstellt

1

| Recherchenort | Abschlußdatum der Recherche | Prüfer |
|---|---|---|
| München | 29. Januar 2024 | Schütte, Maya |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 23 19 5912

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-01-2024

| Im Recherchenbericht angeführtes Patentdokument | Datum der Veröffentlichung | Mitglied(er) der Patentfamilie | Datum der Veröffentlichung |
|---|---|---|---|
| US 2011257317 A1 | 20-10-2011 | KEINE | |
| JP S5922950 A | 06-02-1984 | KEINE | |
| JP H0598106 A | 20-04-1993 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20110257317 A1